# EUROPEAN PATENT APPLICATION

(11) **EP 3 025 745 A1**
(43) Date of publication of application: **01.06.2016**
(21) Application number: 15195136.5
(22) Date of filing: 18.11.2015
(51) Int. Cl.: A61M 5/31

(54) **NOZZLE CAP, AND PREFILLED SYRINGE EMPLOYING THE NOZZLE CAP**

(30) Priority: 27.11.2014 JP 2014240167
(71) Applicant: SUMITOMO RUBBER INDUSTRIES LIMITED, Kobe-shi, Hyogo-ken (JP)
(72) Inventor: NAKANO, Hiroaki, Kobe-shi, Hyogo 651-0072 (JP); ISHIDA, Naoyuki, Kobe-shi, Hyogo 651-0072 (JP)
(74) Representative: Manitz, Finsterwald & Partner GbR

(57) **Abstract**

A nozzle cap (1) to be attached to a nozzle of a medical prefilled syringe is provided. The nozzle cap (1) includes a nozzle cap body, and a film (8) provided at least on an inner surface of the nozzle cap body. The film (8) has a liquid contact portion (6) to be possibly brought into contact with a liquid drug contained in the syringe, and a retention portion (7) to be brought into contact with an outer periphery of the nozzle. The liquid contact portion (6) of the film (8) has a greater thickness than the retention portion (7) of the film (8). The nozzle cap (1) for the prefilled syringe is excellent in sealability, and less liable to adversely influence a liquid drug contained in the syringe. A medical prefilled syringe is also provided, which employs the nozzle cap.

## Description

### TECHNICAL FIELD

The present invention relates to a prefilled syringe to be used in medical applications, and a nozzle cap to be used for the prefilled syringe.

### BACKGROUND ART

Syringes prefilled with a liquid drug (prefilled syringes) are used as medical syringes. In recent years, the prefilled syringes are increasingly used, because the prefilled syringes can be easily handled without the need for transferring a liquid drug into the syringe, and medical malpractice such as transfer of a wrong liquid drug into the syringe can be prevented.

Unlike conventional syringes into which a liquid drug sucked up from a vial or other container is transferred immediately before use, the prefilled syringes are required to serve as a container which is kept in contact with the liquid drug for a long period of time.

Such a prefilled syringe typically includes a syringe barrel having a distal portion (nozzle) to which an injection needle is attached, a gasket provided in the syringe barrel, and a nozzle cap which closes the distal portion. The liquid drug is sealed in a space defined between the gasket and the nozzle cap in the syringe barrel. When the liquid drug is to be administered, the nozzle cap is removed from the distal portion, and the injection needle is attached to the distal portion. Then, a plunger is connected to the gasket, and pushed toward the distal portion to slide the gasket toward the distal portion. Thus, the liquid drug is administered.

The nozzle cap to be used for the syringe is generally made of a crosslinked rubber. It is known that the crosslinked rubber contains various crosslinking components, and these crosslinking components and their thermally decomposed products are liable to migrate into the liquid drug when the liquid rug is kept in contact with the nozzle cap. It is also known that these migrating components adversely influence the efficacy and the stability of some liquid drug.

From this viewpoint, a so-called laminated cap is known, which includes a rubber nozzle cap having a surface laminated with a film having excellent chemical slidability. By covering a liquid contact portion of the rubber nozzle cap with the film, the components of the crosslinked rubber are prevented from migrating into the liquid drug.

Exemplary materials for the film to be used for this purpose include ultrahigh molecular weight polyethylenes and fluororesins which are highly chemically stable. Of these materials, the fluororesins are preferred because of their high slidability and chemical stability. Of these fluororesins, polytetrafluoroethylenes (PTFE) are particularly preferred because of their very high slidability and stability.

On the other hand, the rubber nozzle cap is required to firmly engage with the syringe (the nozzle of the syringe) to seal the liquid drug during storage, and to be easily detached from the nozzle for use. Therefore, the rubber nozzle cap is required to have rubber elasticity for proper engagement, and to have slidability for easy detachment.

However, the film to be used for the aforementioned purpose does not have rubber elasticity and, therefore, impairs the rubber elasticity of the inside crosslinked rubber. The rubber elasticity of the nozzle cap is essential for reliable sealing of the liquid drug. If the nozzle cap has insufficient rubber elasticity, leakage of the inside liquid drug will result. The film preferably has a smaller thickness so as not to impair the rubber elasticity of the inside crosslinked rubber, but a thinner film will fail to sufficiently prevent the migration of the components of the crosslinked rubber into the liquid drug. Further, the PTFE films are generally formed by a skiving method. If the film thickness is small, therefore, pinholes are more likely to occur in the film. The presence of the pinholes makes it impossible to prevent the migration of the components of the crosslinked rubber into the liquid drug.

### CITATION LIST

### PATENT DOCUMENT

Patent Document 1: JP-2013-103451

### SUMMARY OF THE INVENTION

### PROBLEM TO BE SOLVED BY THE INVENTION

It is an object of the present invention to provide a nozzle cap which can reliably seal a liquid drug and prevent components of a rubber from migrating into the liquid drug from a liquid contact portion thereof, and can be smoothly detached from a syringe nozzle.

It is another object of the present invention to provide a medical prefilled syringe employing the nozzle cap.

### SOLUTION TO PROBLEM

According to the present invention, there is provided a nozzle cap to be attached to a nozzle of a medical prefilled syringe, the nozzle cap including a nozzle cap body, and a film provided at least on an inner surface of the nozzle cap body, the film having a liquid contact portion to be possibly brought into contact with a liquid drug contained in the syringe and a retention portion to be brought into contact with an outer periphery of the nozzle, wherein the liquid contact portion of the film has a greater thickness than the retention portion of the film.

According to the present invention, the thickness of the liquid contact portion of the film is not less than 1.2 times the thickness of the retention portion of the film.

According to the present invention, the film is preferably a fluororesin film.

According to the present invention, the film may be a film made of one of a polytetrafluoroethylene (PTFE) resin, a modified PTFE resin and an ethylene tetrafluoroethylene (ETFE) resin.

According to the present invention, there is also provided a medical prefilled syringe to which the nozzle cap is attached.

### EFFECTS OF THE INVENTION

The nozzle cap for the prefilled syringe is excellent in sealability and less liable to adversely influence the liquid drug contained in the syringe. The medical prefilled syringe employs the nozzle cap.

### BRIEF DESCRIPTION OF THE DRAWING

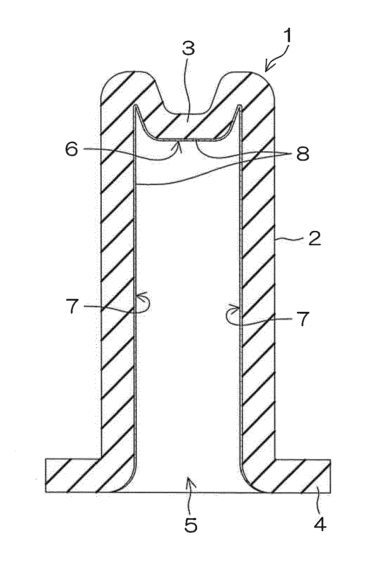

FIG. 1 is a sectional view of a nozzle cap according to one embodiment of the present invention.

### EMBODIMENT OF THE INVENTION

With reference to the attached drawing, one embodiment of the present invention will hereinafter be described specifically.

FIG. 1 is a sectional view of a nozzle cap 1 according to the embodiment of the present invention. The nozzle cap 1 includes a hollow cylindrical peripheral wall 2, an end wall 3 closing one of opposite ends (upper end in FIG. 1) of the peripheral wall 2, and a flange 4 projecting outward from the other end (lower end in FIG. 1) of the peripheral wall 2. The peripheral wall 2, the end wall 3 and the flange 4 are unitarily formed of a rubber material to define a nozzle cap body. The nozzle cap 1 further includes a film 8 provided on an inner surface of the nozzle cap body thereof. The film 8 includes a liquid contact portion 6 present on an inner surface portion of the end wall 3, and a retention portion 7 present on an inner surface portion of the peripheral wall 2.

The nozzle cap 1 is attached to a nozzle projecting from a distal end of a prefilled syringe (not shown) by inserting the nozzle from a lower end inlet 5 of the nozzle cap 1 (as seen in FIG. 1) and bringing a distal end of the nozzle into press contact with the liquid contact portion 6 on the inner surface portion of the end wall 3 of the nozzle cap 1.

With the nozzle cap 1 attached to the nozzle, the retention portion 7 on the inner surface portion of the peripheral wall 2 of the nozzle cap 1 resiliently retains an outer periphery of the nozzle.

In this embodiment, the end wall 3 projects inwardly downward from an upper edge of the peripheral wall 2 to define an upper end wall 3 located slightly below the upper edge of the peripheral wall 2. The end wall 3 is not necessarily required to have such a shape, but merely required to close the one end (upper end) of the peripheral wall 2.

The nozzle cap 1 according to this embodiment is a laminated nozzle cap 1 with its inner surface laminated with the film 8. The liquid contact portion 6 of the film 8, which is to be brought into contact with a liquid drug, has a greater thickness than the retention portion 7 of the film 8 which is to be brought into contact with the nozzle outer periphery.

In order to prevent the components of the crosslinked rubber from migrating into the liquid drug, it is more preferred that the liquid contact portion 6 of the lamination film has a greater thickness. As the thickness of the lamination film increases, the migration of the components of the crosslinked rubber into the liquid drug can be more efficiently prevented. On the other hand, a retention force with which the nozzle cap 1 retains the nozzle outer periphery depends upon the rubber elasticity. Therefore, it is more preferred that the film 8 which does not have the rubber elasticity has a smaller thickness. However, the presence of the lamination film is essential for proper slidability of the nozzle cap 1.

Therefore, the liquid contact portion 6 of the film 8 preferably has a greater thickness than the retention portion 7 of the film 8.

The liquid contact portion 6 is herein defined as a portion of the film 8 present on the inner bottom surface portion of the end wall 3 of the nozzle cap 1 to be brought into contact with the liquid drug.

The retention portion 7 is herein defined as a portion of the film 8 present on the inner surface portion of the peripheral wall 2 of the nozzle cap 1 to be brought into contact with the nozzle peripheral surface.

The absolute thickness values may be properly determined according to characteristic properties required for the product of the nozzle cap 1. Where it is desirable to more reliably prevent the migration of the components of the rubber, the thickness of the film may be entirely increased. Where it is desirable to ensure more reliable sealing of the liquid drug, the thickness of the film may be entirely reduced. Even in these cases, the prevention of the migration of the components of the rubber and the reliable sealing of the liquid drug can be achieved by allowing the film 8 to have a greater thickness in the liquid contact portion 6 than in the retention portion 7.

As described above, the liquid contact portion 6 preferably has a greater thickness for more efficient prevention of the migration of the components of the crosslinked rubber. The liquid contact portion 6 preferably has a thickness of greater than 25 µm, more preferably greater than 35 µm. The upper limit of the thickness is not particularly specified, but preferably not greater than 150 µm for moldability and economy.

As described above, the retention portion 7 preferably has a smaller thickness. The retention portion 7 preferably has a thickness of not greater than 25 µm, more preferably not greater than 20 µm. However, the presence of the lamination film is essential for the slidability. Therefore, the retention portion 7 preferably has a thickness of not less than 5 µm for uniformity thereof.

The type of the film 8 is not particularly specified, as long as the film is capable of preventing the migration of the components of the crosslinked rubber and has more excellent slidability than the rubber, i.e., has a smaller friction coefficient than the rubber. Exemplary materials for the film 8 include ultrahigh molecular weight polyethylenes and fluororesins which are often used for medical applications. Of these materials, the fluororesins are preferred because of their excellent slidability and excellent surface chemical stability. Known fluorine-containing resins are usable as the fluororesins, and specific examples of the fluororesins include PTFE, modified PTFE, ethylene tetrafluoroethylene copolymers (ETFE) and perfluoroalkyl ether polymers (PFA). PTFE and modified PTFE are particularly preferred because of their excellent slidability and chemical stability. ETFE is preferred because of its excellent resistance to γ-ray sterilization. The film may have a single layer structure or a multi-layer structure, as long as the film satisfies the aforementioned thickness requirement.

The material for the nozzle cap body is not particularly specified, but examples of the material for the nozzle cap body include thermosetting rubbers and thermoplastic elastomers. Of these materials, the thermosetting rubbers are preferred because of their excellent heat resistance. Of the thermoplastic elastomers, dynamically crosslinkable thermoplastic elastomers having crosslinking sites are more preferred. The polymer components of these materials are not particularly specified, but ethylene-propylene-diene rubbers and butadiene rubbers excellent in moldability and butyl rubbers, chlorinated butyl rubbers and brominated butyl rubbers excellent in gas permeation resistance are preferred.

The thickness of a portion of the film other than the liquid contact portion 6 and the retention portion 7 is not particularly limited, but may be determined in consideration of the moldability and the economy. The thickness is preferably not less than 5 µm and not greater than 150 µm.

Where the liquid drug to be contained in the prefilled syringe is adversely influenced by neither silicone oil nor curable silicone, the nozzle outer periphery or the inner peripheral surface of the nozzle cap may be coated with common silicone oil or curable silicone to be imparted with excellent slidability.

### EXAMPLES

Plural types of material films and an unvulcanized rubber sheet were prepared, and nozzle cap products of Examples 1 to 7 and Comparative Examples 1 to 3 shown in Table 1 were produced by vulcanization-molding the rubber sheet into a cap shape as shown in FIG. 1. The thickness of the film 8 of each of the nozzle cap products was measured by a method to be described later. The nozzle cap products each had an inner diameter of 9.85 mm.

The material films each had different thicknesses in portions thereof later serving as the liquid contact portion 6 and the retention portion 7, whereby the films of the nozzle cap products each had different thicknesses in the liquid contact portion 6 and in the retention portion 7.

### [Production Method]

### <Fluororesins>

PTFE film (VALFLON (registered trade name) available from Nippon Valqua Industries, Ltd.)
Modified PTFE film (VALFLON Exl (registered trade name) available from Nippon Valqua Industries, Ltd.)
ETFE film (Fluon ETFE (registered trade name) available from Asahi Glass Co., Ltd.)

### <Fluororesin Dispersion>

POLYFLON D210-C (registered trade name) available from Daikin Industries, Ltd.)

The material films were each treated by roughening a surface of the film. Then, the rubber sheet was superposed on the surface of the film, and vulcanization-molded to be thereby bonded to the film (by a method disclosed in JP4908617). The thicknesses of the material films are shown in Table 1.

### <Unvulcanized Rubber Sheet>

Halogenated butyl rubber sheet

### <Crosslinking Agent>

2-di-n-butylamino-4,6-dimercapto-s-triazine, ZISNET DB (registered trade name) available from Sankyo Kasei Co., Ltd.

### [Production Conditions]

Vulcanization temperature: 180°C

Vulcanization time: 8 minutes

Treatment Pressure: 20 MPa

The inert material films each having different thicknesses in different portions thereof were produced in the following manner.

### <PTFE Film>

The PTFE aqueous dispersion was applied onto one surface of the aforementioned PTFE film, and then preheated at 90°C for 5 minutes to evaporate water away from the resulting dispersion film. In turn, the resulting PTFE film was burnt at 350°C for 5 minutes. This operation was repeated a plurality of times, whereby the material film was formed as having desired thicknesses. In order to impart different portions of the material film with different thicknesses, the dispersion was applied or not applied to a portion of the film later serving as the liquid contact portion of the nozzle cap product. Thus, the PTFE film was produced as the material film having different thicknesses in different portions thereof.

### <Modified PTFE Film>

The aforementioned modified PTFE film was used as a base material, and a material film was produced as having different thicknesses in different portions thereof by applying or not applying the PTFE aqueous dispersion on different portions of the modified PTFE film.

### <ETFE Film>

A material film was produced as having different thicknesses in different portions thereof by pressing the aforementioned ETFE film at 300°C between two metal plates properly spaced from each other for thickness control of the material film.

### [Test Method]

### <Measurement of Film Thickness>

The nozzle cap products were each cut, and sections thereof were observed at a magnification of 150X by means of a scanning electron microscope (S-3400N available from Hitachi High-Technologies Corporation) for measurement of the thickness of the film. More specifically, the thickness of the liquid contact portion (a center portion of the bottom of the cap) and the thickness of the retention portion (to be brought into contact with the syringe barrel) were measured. The measurement was performed on five samples for each of the nozzle cap products, and the measured thickness values were averaged. The average thickness is shown in Table 1.

### <Pinhole Test>

The liquid contact portions of 20 samples for each of the nozzle cap products were observed at five points (five visual fields) with a 300X objective lens by means of a video microscope (DVM5000 available from Leica Microsystems Cms Gmbh), and the number of pinholes each having a diameter of not less than 10 µm was determined. The number of the pinholes is shown in Table 1. A nozzle cap product having a pinhole number of not greater than 2 was rated as acceptable.

### <Nozzle Cap Retainability>

After the nozzle cap products were each attached to a syringe barrel having a nominal volume of 100 mL, water was poured to a volume half the nominal volume in the syringe barrel, which was in turn closed with a gasket. Then, the internal pressure of the syringe barrel was increased by squeezing the gasket from the bottom of the syringe barrel, and a test force applied when the nozzle cap was detached from the syringe barrel was measured. For the measurement, a tester (AUTOGRAPH AG-1000D available from Shimadzu Corporation) was used, and the squeezing rate was 50 mm/min. The measurement was performed on 5 samples for each of the nozzle cap products, and the measured force values were averaged. A nozzle cap product having a force value of not less than 400 N was rated as acceptable.

### [Test Results]

The nozzle cap products of Examples 1 to 7 in which the liquid contact portion had a greater film thickness than the retention portion were substantially free from leaching of the components of the crosslinked rubber with a smaller number of pinholes, and excellent in nozzle retainability. The nozzle cap products of Comparative Examples 1 to 3 in which the liquid contact portion had a smaller film thickness than the retention portion each suffered from significant leaching of the components of the crosslinked rubber with a greater number of pinholes, and each had a lower nozzle periphery retention force.

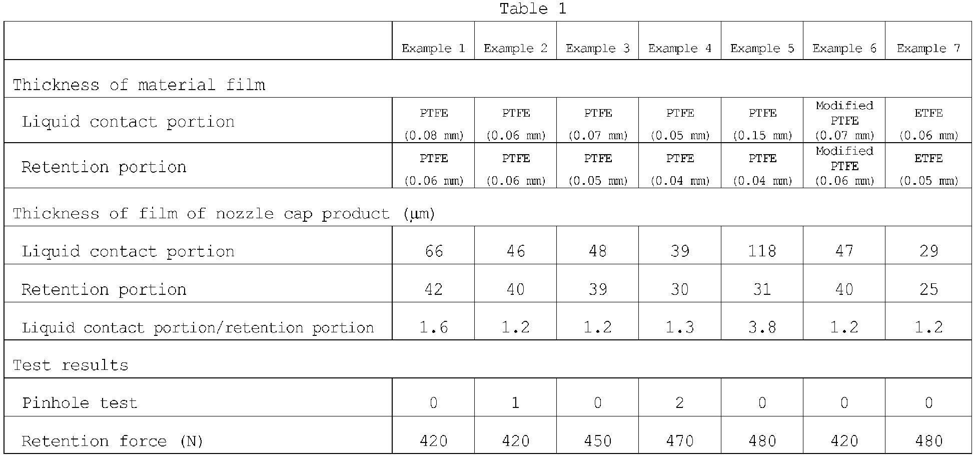

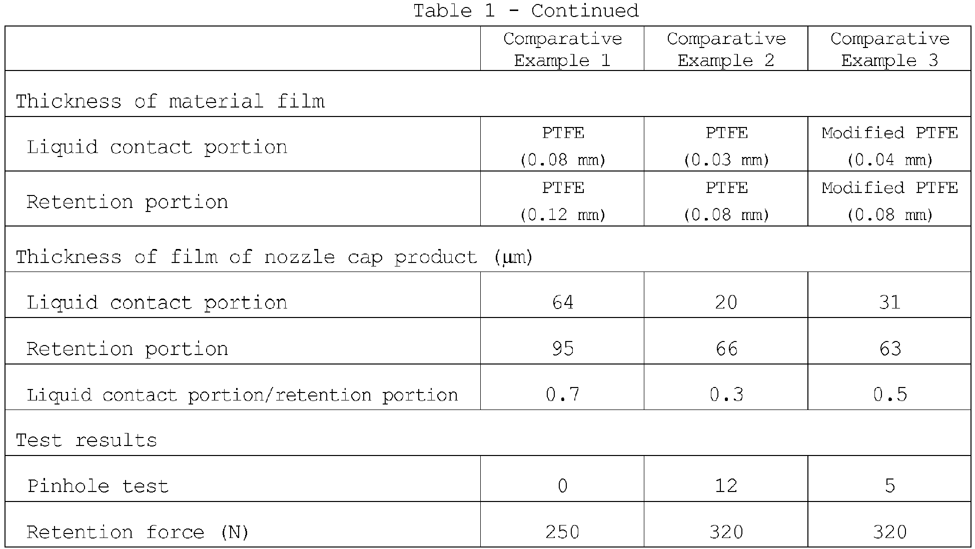

This application corresponds to Japanese Patent Application No. 2014-240167 filed in the Japan Patent Office on November 27, 2014, the disclosure of which is incorporated herein by reference in its entirety.

## Claims

1. A nozzle cap (1) to be attached to a nozzle of a medical prefilled syringe, the nozzle cap (1) comprising:
a nozzle cap body; and
a film (8) provided at least on an inner surface of the nozzle cap body (1), the film having a liquid contact portion (6) to be possibly brought into contact with a liquid drug contained in the syringe and a retention portion (7) to be brought into contact with an outer periphery of the nozzle;
wherein the liquid contact portion (6) of the film has a greater thickness than the retention portion (7) of the film.

2. The nozzle cap according to claim 1, wherein the thickness of the liquid contact portion (6) of the film is not less than 1.2 times the thickness of the retention portion (7) of the film.

3. The nozzle cap according to claim 1 or 2, wherein the film (8) is a fluororesin film.

4. The nozzle cap according to claim 3, wherein the film (8) is a film made of one of a polytetrafluoroethylene (PTFE) resin, a modified PTFE resin and an ethylene tetrafluoroethylene (ETFE) resin.

5. A medical prefilled syringe to which the nozzle cap (1) according to one of claims 1 to 4 is attached.
